# EUROPEAN PATENT SPECIFICATION

(11) **EP 1 008 359 B1**
(45) Date of publication and mention of the grant of the patent: **22.09.2004**
(21) Application number: 98935300.8
(22) Date of filing: 31.07.1998
(51) Int. Cl.: A61M 5/24, A61M 5/31

(54) **MEDICAL CONTAINER**
MEDIZINISCHER BEHÄLTER
CONTENANT MEDICAL

(30) Priority: 01.08.1997 JP 20814697
(43) Date of publication of application: 14.06.2000
(73) Proprietor: TERUMO KABUSHIKI KAISHA, Tokyo 151-0072 (JP)
(72) Inventor: IMAI, Masaomi, Terumo Kabushiki Kaisha, Kanagawa 259-0151 (JP)
(74) Representative: Dronne, Guy
(86) International application number: PCT/JP1998/003415
(87) International publication number: WO 1999/006089

(56) References cited:
- WO-A-99/17820
- JP-A- 7 124 256
- JP-A- 9 173 450
- JP-Y- 52 055 913
- US-A- 5 279 606
- US-A- 5 512 054
- PATENT ABSTRACTS OF JAPAN vol. 1999, no. 03, 31 March 1999 (1999-03-31) & JP 10 337321 A (TERUMO CORP), 22 December 1998 (1998-12-22)

## Description

The present invention relates to a medical container in which a drug or the like is pre-filled.

### Prior Art

To administer a powdery injection to a patient, it is necessary to make a preparation of adding a dissolving liquid to a vial or an ampoule to dissolve the injections therein sufficiently and suck the solution into a syringe. However, the operation is complicated and further, foreign matters may be included in the solution. Further, because a sterile operation should be performed in an open state, the operation necessitates an operator to pay close attention. To solve such a problem, a drug-mixing syringe, wherein a concentrated drug is pre-filled is described in document US 5,522,804. Before administering an injection to a patient, the operator sucks a diluent into the syringe to dilute said concentrated drug. However, the step of diluting the concentrated drug takes time, and further, foreign matters may still be included in the solution. Further, this drug-mixing syringe has a complicated mechanism so that it is quite expensive to manufacture.

Another known device which tends to solve the above-mentioned problem is a kit having a syringe in which a drug is pre-filled is known. However, in the conventional medical container of the type having the syringe in which the drug is pre-filled, a plunger is forced out from the syringe, depending on the capacity of the drug preserved in the syringe. Therefore, the entire kit is large (long) and thus has problems in accommodation and storing performances. Because of the disadvantage, the conventional medical container is used in only a limited range although it has many advantages.

### Disclosure of the Invention

In view of the above-described problems, it is an object of the present invention to provide a medical container having preferable accommodating and storage performances.

The problems can be solved by the present invention described below:
(1) A medical container including :
   an outer cylinder having a pre-sealed nozzle at a front end thereof and an opening at a rear end thereof;
   an inner cylinder open at both ends thereof, inserted into the outer cylinder from a front-end side thereof through the opening of the outer cylinder from a front-end side thereof through the opening of the outer cylinder, and having an outer surface airtightly slidable in contact with an inner surface of the outer cylinder;
   an inner-cylinder-inside-gasket provided inside the inner cylinder and airtightly slidable from the vicinity of the rear end to the front end of the inner cylinder, in contact with an inner surface of the inner cylinder;
   a locking means for preventing said inner-cylinder-inside-gasket from moving toward a rear-end side when said inner-cylinder-inside-gasket is located at a position in the vicinity of said front end of said inner cylinder; and a space airtightly formed in the range from the pre-sealed nozzle to the second gasket, said inner-cylinder-inside-gasket moving from the vicinity of the rear end to the front end of the inner cylinder such that the pressure in said space (7) balances with the pressure outside the medical container (1), so that the volume in said space (7) is kept substantially constant.
(2) A medical container according to above (1), wherein the front end of the inner cylinder is disposed in the vicinity of the front end of the outer cylinder, and the inner-cylinder-inside-gasket is disposed at a rear-end side position spaced at a certain interval from the front end of said inner cylinder.
(3) A medical container according to above (2), wherein a drug is pre-filled in a space formed with an inner surface of the outer cylinder, the inner-cylinder-inside-gasket, and a surface of the inner cylinder.
(4) A medical container according to above (3), wherein said drug is a solution.
(5) A medical container according to any one of above (1) through (4), wherein a locking means for preventing the inner-cylinder-inside-gasket from moving toward a rear-end side is provided in the vicinity of the front end of the inner cylinder.
(6) A medical container according to any one of above (1) through (5), wherein a locking means for preventing the inner-cylinder-inside-gasket from moving toward a front-end side is provided in the vicinity of the front end of the inner cylinder.
(7) A medical container according to any one of above (1) through (6), wherein a slide of the outer surface of the inner cylinder with respect to the inner surface of the outer cylinder is made by a slide of an annular gasket provided to the front end of the inner cylinder or an outer surface in the vicinity of the front end of the inner cylinder with respect to the inner surface of the outer cylinder.
(8) A medical container according to any one of above (1) through (6), wherein a slide of the outer surface of the inner cylinder with respect to the inner surface of the outer cylinder is made by a slide of the outer surface of the inner cylinder with respect to an annular gasket provided to the rear end of the outer cylinder or an inner surface in the vicinity of the rear end of the outer cylinder.
(9) A medical container according to any one of above (1) through (6), wherein a slide of the outer surface of the inner cylinder with respect to the inner surface of the outer cylinder is made through a fine rough surface formed on both the outer surface of the inner cylinder and the inner surface of the outer cylinder.

The medical container of the present invention looks similar to the generally utilized syringe comprising an outer cylinder and a plunger having a gasket installed at the front end thereof. However, the portion of the medical container corresponding to the plunger is cylindrical. A content such as a drug consisting of preferably a solution can be filled into the space formed with the inner cylinder and the inner-cylinder-inside-gasket provided inside the inner cylinder. The space is formed inside the outer cylinder. The space is continuous with other portions inside the outer cylinder such as a front-end portion forward from the front end of the inner cylinder. Accordingly, the same drug can be filled into the space. It is preferable that the outer cylinder and the inner cylinder are cylindrical. When the inner cylinder is moved toward the rear end of the medical container, similarly to the manner of drawing the plunger of the ordinary syringe rearward, the inner-cylinder-inside-gasket moves toward the front end of the inner cylinder such that the pressure in the space balances with the pressure outside the medical container. Consequently, the volume in the space is kept constant.

When the inner cylinder is moved toward the rear end of the medical container, the inner-cylinder-inside-gasket moves toward the front end of the inner cylinder and arrives at a predetermined position of the inner cylinder. In this manner, this operation is completed. It is preferable to make the configuration of the front end of the inner-cylinder-inside-gasket complementary with that of the inner surface of the front-end portion of the inner cylinder. This is because when the inner-cylinder-inside-gasket is moved to the position at which the inner-cylinder-inside-gasket projects forward beyond the front-end portion of the inner cylinder, the content can be discharged thoroughly in a discharging operation which will be described later.

The inner-cylinder-inside-gasket is provided with the function of balancing the pressure in the space with the external pressure, as described above. Thus, the content inside the inner cylinder is moved to the front-end portion of the outer cylinder by the movement of the inner-cylinder-inside-gasket to allow the inner-cylinder-inside-gasket which has moved to the front end of the inner cylinder to be placed in a state in which the inner-cylinder-inside-gasket can discharge the content In this state, the pre-sealed nozzle installed on the front end of the outer cylinder is opened. Then, the inner cylinder is moved toward the front end of the outer cylinder so as to discharge it from the medical container for injection or mixing of the content. To allow the inner-cylinder-inside-gasket to operate in this manner, it is important for the space to be kept airtight. The degree of airtightness required in the present invention should be such that penetration of a gas or a liquid into the space from the outside does not substantially prevent the forward movement of the inner-cylinder-inside-gasket to the predetermined position in the front-end portion of the inner cylinder, which has the operation of keeping the pressure in the space constant. If the content of the medical container is susceptible to influence of the environment at sterilizing time or during storage, it is preferable that the material of each component part of the medical container of the present invention is low or nil in its vapor permeability and gas permeability and that the gap between the inner-cylinder-inside-gasket and the inner cylinder has a high degree of airtightness or the medical container of the present invention is packed with a packing material low or nil in its vapor permeability and gas permeability. If the content of the medical container is susceptible to oxygen, a deoxidizing agent is used. If the content of the medical container is susceptible to moisture, a moisture absorption agent is used. The deoxidizing agent or the moisture absorption agent is provided in an inner or intermediate layer of the packing material or accommodated in the packing material or the component parts of the medical container of the present invention such as the inner-cylinder-inside-gasket. In this manner, the preservability of the content can be improved.

Although the nozzle of the medical container of the present invention is pre-sealed, the sealing can be made by adopting any of known means: for example, the medical container is so constructed that the front end of the nozzle has a self-sealing construction (in this construction, it is preferable to provide nozzle with annular thin fragile portion and a member for opening such as a turn-off member disposed forward from the fragile portion); a film made of rubber or the like is bonded to the front-end portion of the nozzle with an adhesive agent or the like; or a cap is installed on the nozzle by means of taper fitting or screw fitting. In the case where the nozzle is sealed with the cap, it is preferable that the front-end surface of the cap is pierceably formed of rubber or the like. In this case, the nozzle can be connected with a hub of an injection needle by removing the cap from the nozzle, and in addition, the nozzle can be connected with a double-sided needle, with the cap installed on the nozzle. The medical container of the present invention is used such that the needle is connected with the nozzle pre-sealed with the cap or the like by removing the cap from the nozzle to inject a solution to a patient or the nozzle is connected with a connector provided on a three-way stopcock or the like of a transfusion line to carry out mixing of solutions. When the front end of the nozzle and that of the cap are sealed with a pierceable member such as a rubber film, the double-sided needle is connected with the nozzle to introduce the solution into a mixing port of a transfusion bag or that of the transfusion line.

In discharging the content of the medical container by pressing the inner cylinder, an internal pressure is applied to the surface forming the space in which the content has been filled. If the sliding resistance of the inner-cylinder-inside-gasket is lower than the internal pressure, the inner-cylinder-inside-gasket may be moved toward the rear end of the inner cylinder from a position in the vicinity of the front end of the inner cylinder. To prevent this, it is necessary to take a measure for preventing the inner-cylinder-inside-gasket from moving rearward. To this end, it is possible to use selective bonding of the inner-cylinder-inside-gasket to or engaging thereof with a part of the inner surface of the inner cylinder in the vicinity of the front end thereof; provide an engaging means on the inner cylinder; use a convex and concave fitting between the inner cylinder and the inner-cylinder-inside-gasket; or form a small-diameter portion on the front-end portion of the inner cylinder to fit the inner-cylinder-inside-gasket into the small-diameter portion.

In the medical container of the present invention, to slide the outer surface of the inner cylinder in contact with the inner peripheral surface of the outer cylinder, any of known means can be adopted, provided that the space is airtight as described previously: for example, an annular gasket is provided to the front end of the inner cylinder or on the outer surface in the vicinity of the front-end thereof; the annular gasket is provided to the rear end of the outer cylinder or on the inner peripheral surface in the vicinity of the rear end thereof; or the annular gasket is provided on both the inner cylinder and the outer cylinder. In the case where the annular gasket is provided on both the inner cylinder and the outer cylinder, the annular gasket provided on the outer cylinder can protect the inner peripheral surface of the outer cylinder from exposure. In this case, the sliding resistance of the annular gasket provided on the outer cylinder may affect the operability of the medical container. Thus, it is necessary to take care so that the sliding resistance is not high. In the case where the outer cylinder and the inner cylinder are made of glass or the like, a fine rough surface may be formed on a sliding portion of each of the inner peripheral surface of the outer cylinder and the outer surface of the inner cylinder to secure airtight sliding of the inner cylinder on the outer cylinder by the sliding contact there between.

The material of the component parts constituting the medical container of the present invention will be described below. As the material of the outer cylinder (including nozzle) and the inner cylinder, inorganic materials such as glass, metal, and ceramics and synthetic resin are used. It is necessary that the materials is not deformed by an external pressure and so rigid as not to prevent the movement of the inner-cylinder-inside-gasket when the inner cylinder is moved toward the rear end of the medical container. It is preferable that transparent materials are used because the content of the medical container can be checked. It is favorable to select a material from among thermoplastic synthetic resin because the material is required to be rigid and transparent and in addition moldable. It is more favorable to select a material that can be formed by injection molding in addition to the above-described required properties. For example, thermoplastic resin such as polyolefin such as polyethylene, polypropylene, polybutene, polymethylpentene-1, polybutadiene, and cyclic polyolefin; olefin copolymer such as ethylene- α -olefin copolymer; ethylene-vinyl alcohol copolymer, elastomer such as olefin group elastomer and styrene group elastomer; polyester such as polyethylene terephthalate, polyethylene naphthalate and polybutylene terephthalate; vinyl resin such as polyvinyl chloride, polyvinyl alcohol, polyvinyl acetal, polyvinyl acetate, and ethylene-vinyl acetate copolymer; polyvinylidene chloride, polystyrene, polyamide, polyimide, polyurethane, polysulfone, polyamide-imide, polyallylsulfone, polybenzimidazole, polycarbonate, polyether, methacrylic resin, polyphenylene sulfide, cellulose plastic, allyl resin, epoxy resin, phenol resin, nylon, and polyacetal; mixtures of these substances or laminates thereof. Regarding the properties of the above-described materials, those having a water vapor permeation rate at less than 5g·mm/m²/24hr are preferably used for the content such as a medicine or solution to be filled into the medical container is moisture-absorptive or hydrolytic; and those having an oxygen permeation rate at less than 100ml·mm/m²/24hr·atm are preferably used for the content which will undergo an oxidative deterioration. It is possible to select a material having both properties, if necessary. The water vapor permeation rate was measured in the condition of 40°C and RH90% by using a water vapor permeability measuring apparatus (L80-4000, manufactured by Rishee Inc.). Using a gas permeability measuring apparatus (GPM-250, manufactured by GL Science Inc.), the oxygen permeation rate was measured by constant-pressure method and gas chromatography.

The material of the inner-cylinder-inside-gasket and the annular gasket is not limited to specific ones, but the following substances are used: especially vulcanized materials of rubber materials such as natural rubber, isoprene rubber, butyl rubber, butadiene rubber, styrene-butadiene rubber, and silicone rubber; thermoplastic elastomer such as styrene group elastomer such as hydrogen-added styrene-butadiene copolymer, hydrogen-added styrene-ethylene-butylene-styrene block copolymer (hereinafter referred to as SEBS); olefin group elastomer such as ethylene- α -olefin copolymer, propylene- α -olefin copolymer; polyurethane group elastomer and polyamide group elastomer; and mixtures of these substances. Considering chemical-resistant property and insolubility in chemicals, butyl rubber, silicone rubber, styrene group elastomer can be preferably used. In the case where the medical container of the present invention is heat-sterilized together with the content thereof, vulcanized material of isoprene rubber, butyl rubber and styrene-butadiene-styrene block copolymer can be preferably used in consideration of heat-resisting property. In the case where the front end of the nozzle and that of the cap are sealed with a pierceable member such as rubber, the above-described materials can be used. In this case, a piercing-resistant material is particularly preferable to prevent the content from leaking from a needle-pierced portion when a double-sided needle is pierced into the pierceable member. The material of the cap can be selected appropriately from among the materials that can be used for the inner cylinder and the outer cylinder. To seal the nozzle with the cap securely, namely, to prevent loosening effectively, it is preferable to select a material having hardness different from that of the nozzle, irrespective of whether the cap is installed on the nozzle by taper fitting or screw fitting.

A lubricant such as silicone oil may be applied to sliding portions such as the inner-cylinder-inside-gasket or/and the inner peripheral surface of the inner cylinder, the annular gasket provided on the inner peripheral surface of the outer cylinder or/and the inner cylinder, and the annular gasket provided on the outer surface of the inner cylinder or/and the outer cylinder. Further, resin such as Teflon having a very low sliding resistance may be laminated on the sliding portion.

The content to be filled into the medical container of the present invention includes solid agents in the form of solid, powder, and granule, liquid transfusion agents, and injections such as an antibiotic agent, a malignant tumor-resistant agent, a allergy-resistant agent, a hormone agent, a metabolic medicine, a chemotherapy agent a blood preparation, a biological preparation, an agent for circulatory organ, an agent for nervous system, an agent for digestive system, a preparation for respiratory organ, a nutritive agent, vitamins, minerals, sugars, and electrolytes. The content to be filled into the medical container also includes a dissolution-assistant agent a stabilizer, a preservative agent, a painless agent an emulsifying agent, and a suspending agent. In addition to so-called medicines, distilled water and solvents are also included.

### Brief Description of the Drawings

Fig. 1 is a sectional view showing an example of a first embodiment of the medical container of the present invention.
Fig. 2 is a sectional view showing the medical container in use shown in Fig. 1.
Fig. 3 is a sectional view showing the medical container after use shown in Fig. 1.
Fig. 4 is a sectional view showing an example of a second embodiment of the medical container of the present invention.
Fig. 5 is a sectional view showing an example of a third embodiment of the medical container of the present invention.
Fig. 6 shows an example of a fourth embodiment of the inner-cylinder-inside-gasket of the medical container of the present invention.
Fig. 7 is a sectional view showing an example of a fifth embodiment of an inner-cylinder-inside-gasket of the medical container of the present invention.

### Best Mode for Implementing the Invention

The medical container of the present invention will be described in detail below with reference to the drawings.

### (First Embodiment)

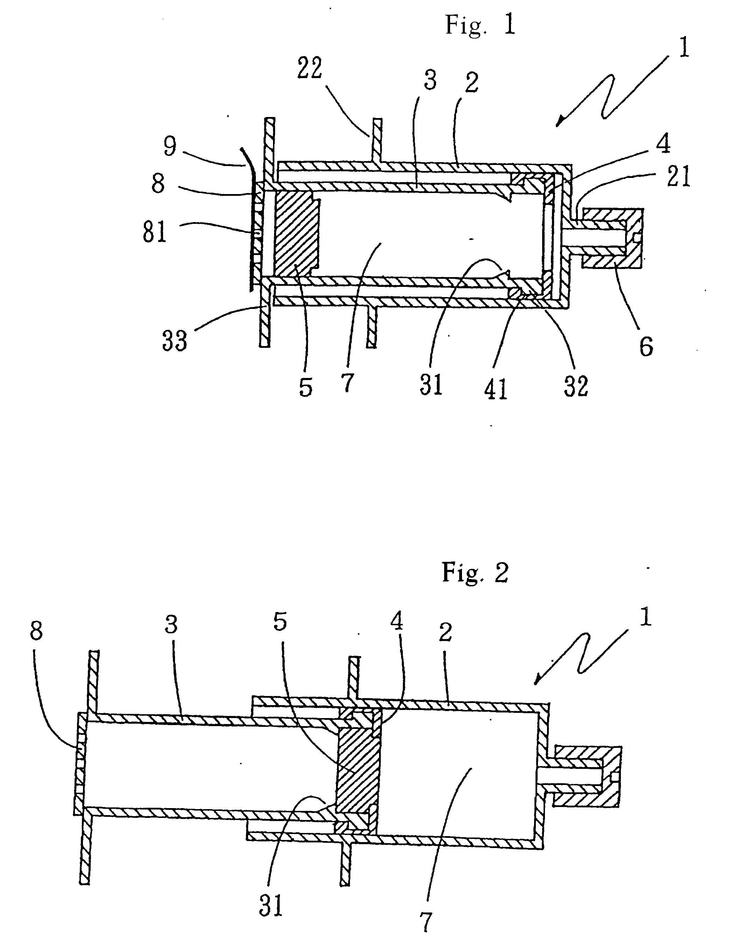

Fig. 1 is a sectional view showing a medical container 1 of the present invention. The medical container 1 includes an outer cylinder 2 made of polypropylene and having a nozzle 21 at its front end and an opening at its rear end; a cap 6 made of butyl rubber and closing the nozzle 21; an inner cylinder 3 made of polypropylene open at its both ends and having a ring-shaped first gasket 4 made of a mixture of SEBS and polypropylene and fitted on a peripheral surface of a front-side opening of the inner cylinder 3; and a second gasket 5 airtightly slidable inside the inner cylinder 3 and made of the same material as that of the first gasket 4. The inner cylinder 3 is inserted into the outer cylinder 2 from the opening at its rear end so that the first gasket 4 is airtightly slidable inside the outer cylinder 2. Although not shown in the drawings, silicone oil is applied to a sliding surface of the first gasket 4 that slides on a sliding surface of the outer cylinder 2 and to a sliding surface of the second gasket 5 that slides on a sliding surface of the inner cylinder 3 to enhance the sliding performance thereof. The front-end side in each drawing indicates the direction in which the nozzle 21 is provided, whereas the rear end side indicates the side opposite to the front-end side.

Fig. 1 shows the state of the medical container 1 before it is used. The front end of the inner cylinder 3 is located in the vicinity of the front end of the outer cylinder 2. The second gasket 5 is located in the vicinity of the rear end of the inner cylinder 3. A space 7 is airtightly formed in the range from the nozzle 21 to the second gasket 5 with the cap 6 sealing the nozzle 21, the first gasket 4, the second gasket 5, the inner surface of the front-end portion of the outer cylinder 2, and the inner surface of the inner cylinder 3. Although not shown in the drawings, a drug is enclosed in the space 7. As the state of the drug, although solution is preferable as described previously, a solid agent such as powder or the like can be used. The volume of the space 7 is kept constant because when a spatial portion at the front-end part of the outer cylinder 2 is increased by moving the inner cylinder 3 to the rear-end side relatively to the outer cylinder 2, the second gasket 5 moves to the front-end side so as to reduce the volume of the spatial portion inside the inner cylinder 3. When the inner cylinder 3 is located at an injecting operation position shown in Fig. 2 by pulling the inner cylinder 3 inserted into the outer cylinder 2 to the rear-end side by utilizing this action, the second gasket 5 inside the inner cylinder 3 moves to the front-end side. As a result of the entire drug present in the space 7, a part thereof inside the inner cylinder 3 can be moved into the outer cylinder 2. In the medical container of the present invention located at the injecting position, the drug can be discharged by pressing the inner cylinder 3 to the front-end side after unsealing the nozzle 21.

In the medical container 1 shown in Fig. 1, a stopper 8 is provided on the opening at the rear-end side of the inner cylinder 3 to prevent the second gasket 5 from projecting out from the inner cylinder 3 and keep a sanitary state. As in the case of the inner cylinder and that of the outer cylinder, the stopper 8 is made of polypropylene. However, the stopper 8 may be made of any of the previously described materials that can be used for the inner cylinder and the outer cylinder. Any other materials may be used for the stopper 8 provided that they can prevent the second gasket 5 from projecting out from the inner cylinder 3 and keep the sanitary state. A vent 81 is formed on the stopper 8 to improve the sliding performance of the second gasket 5. However, in the case where the stopper 8 is formed of an air-permeable material such as mesh, nonwoven cloth or a porous membrane, the stopper 8 is not necessarily provided with the vent 81. The stopper 8 may be formed integrally with the inner cylinder 3. But it is preferable to bond the stopper 8 to the inner cylinder 3 with an adhesive agent or ultrasonic wave, high frequency wave or heating because in manufacturing process, mostly, the second gasket 5 is inserted into the inner cylinder 3 from the opening of the inner cylinder 3 formed at its rear end. To keep a sterile state or a preferable sanitary state, a separable sheet 9 is bonded to the stopper 8 through an adhesive agent Immediately before the use of the medical container 1, the sheet 9 is separated from the stopper 8. The sheet 9 is made of synthetic resin such as olefin group resin such as polyethylene, polypropylene, and the like; ionomer; polyester group resin such as polyethylene terephthalate, polybutylene terephthalate, and the like; polystyrene, poly-(4-methylpentene-1), polyamide and the like; a metal foil such as an aluminum foil; or laminated sheets consisting of these substances.

To improve operability, an annular convex portion 32 is formed on the outer surface of the front-end portion of the inner cylinder 3, and an annular concave portion 41 is formed on the inner peripheral surface of the first gasket 4. The annular convex portion 32 and the annular concave portion 41 fit on each other. Instead, a concave portion may be formed on the inner cylinder 3 to fit a part of the first gasket 4 into the concave portion to thereby fix the inner cylinder 3 and the first gasket 4 to each other. An annular locking portion 31 right-angled triangular in section is formed on the inner surface of the inner cylinder 3 to lock the second gasket 5 thereto when the second gasket 5 moves to the front-end side. The locking portion 31 may have other shapes, provided that it allows the second gasket 5 to move beyond the locking portion 31 when the second gasket 5 moves to the front-end side and has a function of preventing the second gasket 5 from moving to the rear-end side. For example, the annular locking portion 31 may be cut out partly or may have two or more convexities having convex portions formed thereon intermittently. The locking portion 31 has a function of preventing the second gasket 5 from moving to the rear end side. A small-diameter opening is formed at the front end surface of the first gasket 4 to receive penetration of a small-diameter convex portion formed at the front end of the second gasket 5 and having a smaller diameter than the inner diameter of the inner cylinder 3. The small-diameter opening prevents the second gasket 5 from moving to the front-end side beyond it. Gripping portions 22 and 33 having any desired shapes such as the shape of a disk are formed integrally with the outer cylinder 2 and the inner cylinder 3, respectively.

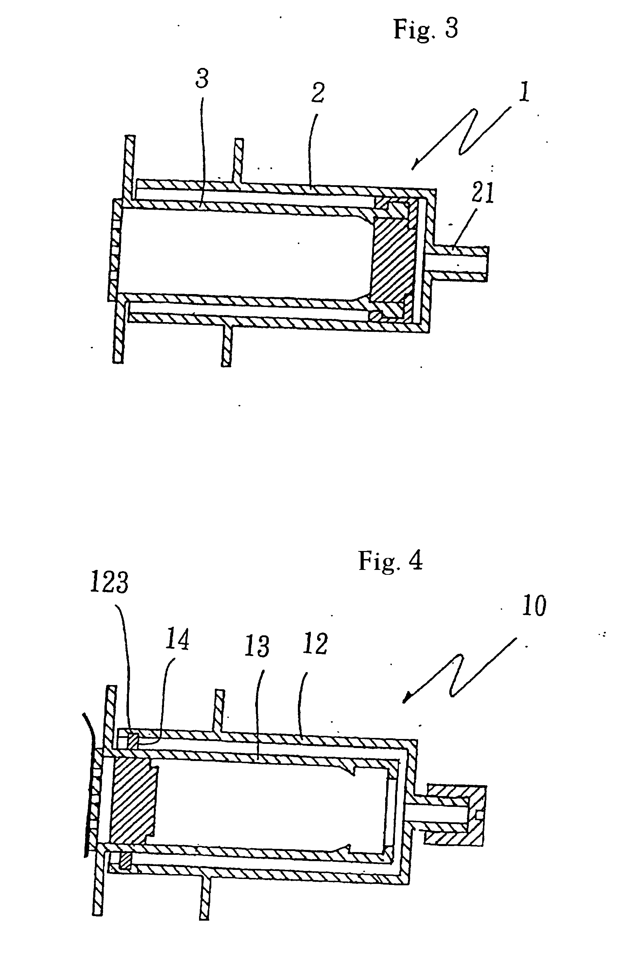

The using method will be described below. In using the medical container shown in Fig. 1 showing the state before use, the sheet 9 is separated from the stopper 8 in the case where the sheet 9 is provided thereon. Then, the inner cylinder 3 is pulled, similarly to the manner of pulling the plunger of an ordinary syringe in performing a sucking operation. Fig. 2 shows the state of the inner cylinder 3 that has been pulled. The space 7 is placed in an airtight state by the sealed nozzle 21, the first gasket 4, and the second gasket 5. Thus, when the inner cylinder 3 is pulled, the second gasket 5 moves to the front-end side relatively to the inner cylinder 3 such that the volume of the space 7 is kept constant. Then, the second gasket 5 is fixed to the front-end portion of the inner cylinder 3 by the locking portion 31 and the front-end surface of the first gasket 4. As shown in Fig. 1 and other figures, the locking portion 31 is right-angled triangular in section and has a descent toward its rear end. Therefore, the second gasket 5 can move to the front-end side beyond the locking portion 31 but cannot move to the rear-end side from the locking portion 31 and receives an internal pressure applied to the wall surface forming the space 7 when the inner cylinder 3 is pressed. With the movement of the second gasket 5, the space 7 is eliminated from the inner cylinder 3 and formed at the front-end side of the outer cylinder 2. By unsealing the nozzle 21, the content such as the unshown drug enclosed in the space 7 can be discharged by pressing the inner cylinder 3, similarly to the manner of discharging the drug or the like sucked into an ordinary syringe by pressing a plunger. Fig. 3 shows the state of the medical container 1 from which the drug has been discharged.

If the content enclosed in the space 7 is a solid such as a powdery agent or is a viscous agent which cannot be easily discharged, the inner cylinder 3 having the second gasket 5 fixed to its front end is pressed into the outer cylinder 2 to some extent Then, the inner cylinder 3 is pulled rearward to suck a dissolving liquid such as water into the outer cylinder 2 to dissolve the powdery agent therein. Then, the inner cylinder 3 is pressed into the outer cylinder 2. In this manner, the drug can be pressed out easily from the nozzle 21.

### (Second Embodiment)

Another embodiment of the present invention will be described below with reference to Fig. 4. The first gasket 4 of the first embodiment is provided at the front end of the inner cylinder 3. However, in a medical container 10 of the second embodiment, a first gasket 14 is provided not on an inner cylinder 13 but on the outer cylinder 12 by fitting it into an annular concave portion 123 formed on the inner surface of the rear-end portion of the outer cylinder 12. Thus, the inner cylinder 13 of the second embodiment is not provided with an annular convex portion corresponding to the annular convex portion 32 of the first embodiment provided on the outer surface of the inner cylinder 3 to fix the first gasket thereto. The constructions of the other parts of the second embodiment are similar to those of the first embodiment The construction of the second embodiment reduces the possibility to a higher extent that the inner surface of the outer cylinder that contacts the drug is polluted therewith.

### (Third Embodiment)

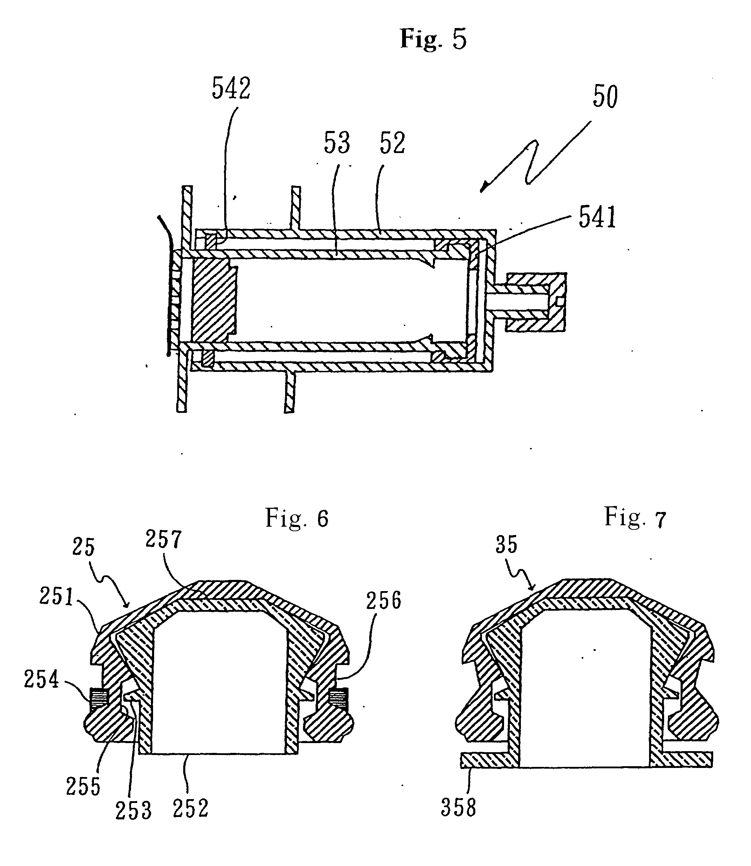

Still another embodiment of the present invention will be described below with reference to Fig. 5. A medical container 50 of the third embodiment has a construction similar to that of the above-described embodiments except that the medical container 50 has the first gasket 4 of the first embodiment and the first gasket 14 of the second embodiment. That is, an inner cylinder-side first gasket 541 is provided at the front-end portion of an inner cylinder 53, similarly to the first embodiment, and an outer cylinder-side first gasket 542 is provided at the rear-end portion of an outer cylinder 52, similarly to the second embodiment Accordingly, the outer cylinder-side first gasket 542 reduces the possibility to a higher extent that the inner surface of the outer cylinder that contacts the drug is polluted therewith and the inner cylinder-side first gasket 541 allows the inner cylinder to be operated stably. The function demanded for the first gasket is shared to both first gaskets as described below. To allow the inner cylinder-side first gasket 541 to have the function of preventing the content from leaking and supporting the inner cylinder stably, allow the outer cylinder-side first gasket 542 to have the function of preventing the inner surface of the outer cylinder from being polluted, and to operate the medical container 50 smoothly, it is preferable to set the sliding resistance of the outer cylinder-side first gasket 542 lower than that of the inner cylinder-side first gasket 541.

### (Fourth Embodiment)

Still another embodiment of the present invention will be described below with reference to Fig. 6. As the inner-cylinder-inside-gasket of the medical container of the present invention, instead of the second gasket 5 that is used in the first through third embodiments, the following inner-cylinder-inside-gasket may be used. In the case where the inner-cylinder-inside-gasket of the fourth embodiment is used, the inner cylinder to be used in the fourth embodiment has a small-diameter portion with a reduced inner diameter at its front-end portion. An inner-cylinder-inside-gasket 25 shown in Fig. 6 has a body 251 made of a mixture of SEBS and polypropylene and a core material 252 made of polypropylene. The core material 252 is provided with an annular flange 253 on the outer peripheral surface thereof in approximately the center in the axial direction thereof such that the annular flange 253 is coincident with an annular concave portion 255 formed on the inner surface of the body 251. A fixing ring 254 made of hard polypropylene and contacting the above-described small-diameter portion is installed on the outer peripheral surface, of the body 251, corresponding to the annular flange 253. An annular concave portion 256 is formed on the outer surface of the body 251 such that the annular concave portion 256 is disposed above (front-end side) the fixing ring 254. The annular concave portion 256 engages the small-diameter portion liquid-tightly by the elasticity of the gasket and the internal pressure in the space.

In the medical container of the present invention using the inner-cylinder-inside-gasket 25, when the inner cylinder accommodated in the outer cylinder moves rearward, an outer surface-diameter-increasing portion of the bottom portion of the inner-cylinder-inside-gasket 25 allows the inner-cylinder-inside-gasket 25 located at the rear-end side of the inner cylinder to move forward inside the inner cylinder, with the space kept airtight. At this time, a space formed between the inside of the inner cylinder and the inside of the outer cylinder has a negative pressure. Consequently, the front-end portion of the body 251 is pulled inward in the space to form a gap in a boundary 257 between the body 251 and the core material 252, and the rear-end portion of the body 251 is pulled by the front end thereof.

As a result, in a forward movement of the inner-cylinder-inside-gasket 25 inside the inner cylinder in a rearward movement of the inner cylinder, the sliding resistance of the outer surface of the inner-cylinder-inside-gasket 25 with respect to the inner cylinder becomes low. Thus, the inner-cylinder-inside-gasket 25 can easily engage the annular convex portion formed at the front end of the inner cylinder. Even though the inner cylinder is moved rearward excessively, the fixing ring 254 contacts the small-diameter portion formed at the front end of the inner cylinder. Thus, the inner-cylinder-inside-gasket 25 can be prevented from being dropped in the space.

When the inner cylinder is pressed, the inner-cylinder-inside-gasket 25 also moves forward. At this time, the annular concave portion 256 formed on the outer surface of the body 251 is in engagement with the small-diameter portion formed at the front end of the inner cylinder. When the inner cylinder is pressed, the space has a positive pressure. Consequently, the front-end portion of the body 251 contracts toward the rear-end side;

At this time, the body 251 is brought into close contact with the front-end portion of the inner cylinder and a part of its inner surface in the vicinity of its front-end portion so as to tightly seal the gap between the inner cylinder and the inner-cylinder-inside-gasket. Thereby, it is possible to reliably prevent the internal pressure in the space generated by the pressing of the inner cylinder from being released from other portions than the nozzle. Thus, it does not occur that the content can be prevented from leaking from other portions than the nozzle.

### (Fifth Embodiment)

Still another embodiment of the present invention will be described below with reference to Fig. 7. An inner-cylinder-inside-gasket 35 of the fifth embodiment is similar to the inner-cylinder-inside-gasket 25 of the fourth embodiment except that the fixing ring 254 is removed from the inner-cylinder-inside-gasket 25 and a bottom annular flange 358 is provided at the rear end of the core material of the gasket. Accordingly, the inner cylinder of the fifth embodiment having a configuration similar to that of the fourth embodiment is used. The bottom annular flange 358 has the same function as that of the fixing ring 254 of the fourth embodiment, thus preventing the drop of the inner-cylinder-inside-gasket.

### Industrial Applicability

The conventional medical container of the type having a syringe in which medicine is pre-filled has a problem that a plunger is forced out from the syringe, depending on the capacity of the medicine. But according to the medical container of the present invention does not have such a problem. That is, the medical container of the present invention has superior accommodating and storing performances before it is used. Because a small space suffices for the storage of the medical container, the medical container is economical. It is superior to the conventional pre-filled syringe, because much medicine can be stored in a limited space. Thus, the medical container is very useful in emergency. Further, the administering operation can be performed very as easily as the conventional pre-filled syringe. The administering method of the present invention is more excellent than the method of sucking medicine into a vacant syringe from an ample or the like.

## Claims

1. A medical container (1) comprising:
an outer cylinder (2) having a pre-sealed nozzle (21) at a front end thereof and an opening at a rear end thereof;
an inner cylinder (3) open at both ends thereof, inserted into said outer cylinder (2) from a front-end side thereof through said opening of said outer cylinder (2), and having an outer surface airtightly slidable in contact with an inner surface of said outer cylinder (2),
an inner-cylinder-inside-gasket (5) provided inside the inner cylinder (3) and airtightly slidable from the vicinity of the rear end to the front end of the inner cylinder, in contact with an inner surface of the inner cylinder (3); and a locking means (31) for preventing said inner-cylinder-inside-gasket (5) from moving toward a rear-end side when said inner-cylinder-inside-gasket (5) is located at a position in the vicinity of said front end of said inner cylinder (3), **characterized in that**;
a space (7) is airtightly formed in the range from the pre-sealed nozzle (21) to the inner-cylinder-inside-gasket (5), and **in that** the inner-cylinder-inside-gasket moves from the vicinity of the rear end to the front end of the inner cylinder such that the pressure in said space (7) balances with the pressure outside the medical container (1), so that the volume in said space (7) is kept constant.

2. A medical container (1) according to claim 1, wherein when said front end of said inner cylinder (3) is disposed in the vicinity of said front end of said outer cylinder (2), said inner-cylinder-inside-gasket (5) is disposed at a rear-end side position spaced at a certain interval from said front end of said inner cylinder (3).

3. A medical container (1) according to claim 2, wherein a drug is pre-filled in a space (7) formed with an inner surface of said outer cylinder (2), said inner-cylinder-inside-gasket (5), and a surface of said inner cylinder (3).

4. A medical container (1) according to claim 3, wherein said drug is a solution.

5. A medical container (1) according to any one of claims 1 through 4, wherein a locking means (31) for preventing said inner-cylinder-inside-gasket (5) from moving toward a rear-end side is provided in the vicinity of said front end of said inner cylinder (3).

6. A medical container (1) according to any one of claims 1 through 5, wherein a locking means (31) for preventing said inner-cylinder-inside-gasket (5) from moving toward a front-end side is provided in the vicinity of said front end of said inner cylinder.

7. A medical container (1) according to any one of claims 1 through 6, wherein a slide of said outer surface of said inner cylinder (3) with respect to said inner surface of said outer cylinder (2) is made by a slide of an annular gasket (4) provided to said front end of said inner cylinder (3) or an outer surface in the vicinity of said front end of said inner cylinder (3) with respect to said inner surface of said outer cylinder (2).

8. A medical container according to any one of claims 1 through 6, wherein a slide of said outer surface of said inner cylinder (3) with respect to said inner surface of said outer cylinder (2) is made by a slide of said outer surface of said inner cylinder (3) with respect to an annular gasket (14) provided to said rear end of said outer cylinder (2) or an inner surface in the vicinity of said rear end of said outer cylinder (2).

9. A medical container according to any one of claims 1 through 6, wherein a slide of said outer surface of said inner cylinder (3) with respect to said inner surface of said outer cylinder (2) is made through a fine rough surface formed on both said outer surface of said inner cylinder (3) and said inner surface of said outer cylinder (2).

## Patentansprüche

1. Medizinischer Behälter (1), der aufweist:
einen äußeren Zylinder (2) mit einer vorab abgedichteten Düse (21) an einem vorderen Ende und einer Öffnung an einem hinteren Ende desselben;
einen inneren Zylinder (3), der an beiden Enden offen ist, und von einer vorderen Seite in den äußeren Zylinder (2) durch die Öffnung des äußeren Zylinders (2) eingeführt ist und eine Außenfläche hat, die in Kontakt mit einer Innenfläche des äußeren Zylinders (2) luftdicht gleiten kann,
eine Innendichtung (5) in dem inneren Zylinder, die in dem inneren Zylinder (3) vorgesehen ist und von der Nähe des hinteren Endes zum vorderen Ende des inneren Zylinders in Kontakt mit einer Innenfläche des inneren Zylinders (3) luftdicht gleiten kann;
und eine Verriegelungseinrichtung (31) um zu verhindern, dass sich die Innendichtung (5) in dem inneren Zylinder in Richtung einer rückwärtigen Seite bewegt, wenn die Innendichtung (5) in dem inneren Zylinder in einer Position in der Nähe des vorderen Endes des inneren Zylinders (3) angeordnet ist, **dadurch gekennzeichnet, dass**
ein Raum (7) luftdicht im Bereich von der vorab abgedichteten Düse (21) zu der Innendichtung (5) gebildet ist, und dass die Innendichtung in dem inneren Zylinder sich von der Nähe des hinteren Endes zu dem vorderen Ende des inneren Zylinders bewegt, so dass der Druck in dem Raum (7) sich mit dem Druck außerhalb des medizinischen Behälters (1) ausgleicht, damit das Volumen in dem Raum (7) konstant gehalten wird.

2. Medizinischer Behälter (1) nach Anspruch 1, wobei, wenn das vordere Ende des inneren Zylinders (3) in der Nähe des vorderen Endes des äußeren Zylinders (2) angeordnet ist, die Innendichtung (5) in dem inneren Zylinder in einer Position auf der rückwärtigen Seite angeordnet ist, die in einem bestimmten Abstand von dem vorderen Ende des inneren Zylinders (3) angeordnet ist.

3. Medizinischer Behälter (1) nach Anspruch 2, wobei ein Medikament vorab in einen Raum (7) gefüllt wird, der mit einer Innenfläche des äußeren Zylinders (2), der Innendichtung (5) in dem inneren Zylinder und einer Fläche des inneren Zylinders (3) gebildet ist.

4. Medizinischer Behälter (1) nach Anspruch 3, wobei das Medikament eine Lösung ist.

5. Medizinischer Behälter (1) nach einem der Ansprüche 1 bis 4, wobei eine Verriegelungseinrichtung (31) in der Nähe des vorderen Endes des inneren Zylinders (3) vorgesehen ist, um zu verhindern, dass sich die Innendichtung (5) in dem inneren Zylinder in Richtung einer rückwärtigen Seite bewegt.

6. Medizinischer Behälter (1) nach einem der Ansprüche 1 bis 5, wobei eine Verriegelungseinrichtung (31) in der Nähe des vorderen Endes des inneren Zylinders vorgesehen ist, um zu verhindern, dass sich die Innendichtung (5) in dem inneren Zylinder in Richtung einer vorderen Seite bewegt.

7. Medizinischer Behälter (1) nach einem der Ansprüche 1 bis 6, wobei ein Gleiten der Außenfläche des inneren Zylinders (3) in Bezug auf die Innenfläche des äußeren Zylinders (2) durch ein Gleiten einer ringförmigen Dichtung (4), die an dem vorderen Ende des inneren Zylinders (3) oder einer Außenfläche in der Nähe des vorderen Endes des inneren Zylinders (3) vorgesehen ist, in Bezug auf die Innenfläche des äußeren Zylinders (2), erfolgt.

8. Medizinischer Behälter nach einem der Ansprüche 1 bis 6, wobei ein Gleiten der Außenfläche des inneren Zylinders (3) in Bezug auf die Innenfläche des äußeren Zylinders (2) durch ein Gleiten der Außenfläche des inneren Zylinders (3) in Bezug auf eine ringförmige Dichtung (14) erfolgt, die an dem hinteren Ende des äußeren Zylinders (2) oder einer Innenfläche in der Nähe des hinteren Endes des äußeren Zylinders (2) vorgesehen ist.

9. Medizinischer Behälter nach einem der Ansprüche 1 bis 6, wobei ein Gleiten der Außenfläche des inneren Zylinders (3) in Bezug auf die Innenfläche des äußeren Zylinders (2) mittels einer fein gerauten Oberfläche erfolgt, die sowohl auf der Außenfläche des inneren Zylinders (3) als auch auf der Innenfläche des äußeren Zylinders (2) gebildet ist.

## Revendications

1. Contenant médical (1) comprenant :
un cylindre extérieur (2) ayant une buse (21) préalablement scellée à son extrémité avant, et une ouverture à son extrémité arrière ;
un cylindre intérieur (3) ouvert à ses deux extrémités, introduit dans ledit cylindre extérieur (2) depuis le côté de son extrémité avant à travers ladite ouverture dudit cylindre extérieur (2), et présentant une surface extérieure capable de coulisser de façon étanche à l'air en contact avec une surface intérieure dudit cylindre extérieur (2),
un joint intérieur au cylindre intérieur (5), prévu à l'intérieur du cylindre intérieur (3) et capable de coulisser de façon étanche à l'air depuis le voisinage de l'extrémité arrière jusqu'à l'extrémité avant du cylindre intérieur, en contact avec une surface intérieure du cylindre intérieur (3) ; et des moyens de blocage (31) pour empêcher ledit joint intérieur (5) de se déplacer vers le côté de l'extrémité arrière quand ledit joint intérieur (5) est situé au voisinage de ladite extrémité avant dudit cylindre intérieur (3), **caractérisé en ce que** :
un espace (7) étanche à l'air s'étend de la buse préalablement scellée (21) au joint intérieur (5), et **en ce que** le joint intérieur se déplace depuis le voisinage de l'extrémité arrière jusqu'à l'extrémité avant du cylindre intérieur de telle façon que la pression dans ledit espace (7) s'équilibre avec la pression à l'extérieur du contenant médical (1), de sorte que le volume dans ledit espace (7) est maintenu constant.

2. Contenant médical (1) selon la revendication 1, dans lequel, lorsque ladite extrémité avant dudit cylindre intérieur (3) est disposée au voisinage de ladite extrémité avant dudit cylindre extérieur (2), ledit joint intérieur (5) est disposé dans une position située du côté de l'extrémité arrière, à un certain intervalle de ladite extrémité avant dudit cylindre intérieur (3).

3. Contenant médical (1) selon la revendication 2, dans lequel un médicament est préalablement rempli dans un espace (7) formé par une surface intérieure dudit cylindre extérieur (2), ledit joint intérieur (5), et une surface dudit cylindre intérieur (3).

4. Contenant médical (1) selon la revendication 3, dans lequel ledit médicament est une solution.

5. Contenant médical (1) selon l'une quelconque des revendications 1 à 4, dans lequel un moyen de blocage (31), destiné à empêcher audit joint intérieur (5) de se déplacer vers le côté de l'extrémité arrière, est prévu au voisinage de ladite extrémité avant dudit cylindre intérieur (3).

6. Contenant médical (1) selon l'une quelconque des revendications 1 à 5, dans lequel un moyen de blocage (31), destiné à empêcher ledit joint intérieur (5) de se déplacer vers le côté de l'extrémité avant, est prévu au voisinage de ladite extrémité avant dudit cylindre intérieur.

7. Contenant médical (1) selon l'une quelconque des revendications 1 à 6, dans lequel le coulissement de ladite surface extérieure dudit cylindre intérieur (3) par rapport à ladite surface intérieure dudit cylindre extérieur (2) est réalisé par coulissement d'un joint annulaire (4) prévu sur ladite extrémité avant dudit cylindre intérieur (3) ou sur une surface extérieure au voisinage de ladite extrémité avant dudit cylindre intérieur (3), par rapport à ladite surface intérieure dudit cylindre extérieur (2).

8. Contenant médical selon l'une quelconque des revendications 1 à 6, dans lequel le coulissement de ladite surface extérieure dudit cylindre intérieur (3) par rapport à ladite surface intérieure dudit cylindre extérieur (2) est réalisé par coulissement de ladite surface extérieure dudit cylindre intérieur (3) par rapport à un joint annulaire (14) prévu sur ladite extrémité arrière dudit cylindre extérieur (2) ou sur une surface intérieure au voisinage de ladite extrémité arrière dudit cylindre extérieur (2).

9. Contenant médical selon l'une quelconque des revendications 1 à 6, dans lequel le coulissement de ladite surface extérieure dudit cylindre intérieur (3) par rapport à ladite surface intérieure dudit cylindre extérieur (2) est réalisé via une surface finement rugueuse formée à la fois sur ladite surface extérieure dudit cylindre intérieur (3) et sur ladite surface intérieure dudit cylindre extérieur (2).
